(19) Europäisches Patentamt
European Patent Office
Office européen des brevets

(11) EP 0 644 189 B1

(12) **EUROPEAN PATENT SPECIFICATION**

(45) Date of publication and mention of the grant of the patent:
**16.01.2002 Bulletin 2002/03**

(51) Int Cl.[7]: **C07D 301/00**, C07D 301/32, C07D 303/02, C07B 57/00

(21) Application number: **94110146.1**

(22) Date of filing: **29.06.1994**

(54) **Resolution of stereoisomers of aliphatic epoxides**

Trennung von stereoisomeren aliphatischer Epoxiden

Résolution de stéréoisomères d'époxydes aliphatiques

(84) Designated Contracting States:
**CH DE FR GB IT LI**

(30) Priority: **17.09.1993 US 122288**

(43) Date of publication of application:
**22.03.1995 Bulletin 1995/12**

(73) Proprietor: **DAICEL CHEMICAL INDUSTRIES, LTD.**
**Sakai-shi, Osaka (JP)**

(72) Inventor: **Tachibana, Kozo**
**West Chester, PA 19380 (US)**

(74) Representative:
**Grünecker, Kinkeldey, Stockmair & Schwanhäusser Anwaltssozietät**
**Maximilianstrasse 58**
**80538 München (DE)**

(56) References cited:
**EP-A- 0 121 776**
**EP-A- 0 157 365**
**EP-A- 0 158 884**
**EP-A- 0 436 722**
**EP-A- 0 569 992**

- **JOURNAL OF CHROMATOGRAPHY, BIOMEDICAL APPLICATIONS, vol.583, no.2, 2 December 1992, AMSTERDAM NL pages 231 - 235 E.H. OLIW 'Enantioselective separation of some polyunsaturated epoxy fatty acids by high-performance liquid chromatography on a cellulose phenylcarbamate (Chiracel OC) stationary phase'**
- **CHEMICAL ABSTRACTS, vol. 112, no. 1, 1 January 1990, Columbus, Ohio, US; abstract no. 775n, T.D. HAMMONDS ET AL. 'Resolution of epoxyeicosatrienoate enantiomers by chiral phase chromatography' page 780 ; & ANAL. BIOCHEM., vol.182, no.2, 1989 pages 300 - 303**
- **ANALYTICAL CHEMISTRY, vol.62, no.9, 1 May 1990, COLUMBUS US pages 914 - 923 D.W. ARMSTRONG ET AL. 'Polar-liquid, derivatized cyclodextrin stationary phase for the capillary gas chromatography separation of enantiomers'**
- **HUHEEY J.E.: 'Anorganische Chemie', 1988, WALTER DE GRUYTER, BERLIN**
- **PIRKLE W.H. AND POCHAPSKY T.C. J. AM. CHEM. SOC. no. 109, 1987, pages 5975 - 5982**
- **J. CHEM. SOC 1952, pages 3940 - 3943**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to a method of resolving stereoisomers of aliphatic epoxides, and especially to the resolution of enantiomeric isomers thereof.

Since one enantiomer of an asymmetric compound can differ from another enantiomer of that asymmetric compound in its actions within a living body, methods capable of providing optical resolution of enantiomeric mixtures as well as asymmetric synthesis methods have been considered important in organic chemistry.

Generally, compounds represented by Formula (1) set forth hereinbelow, are highly reactive and at the same time possess a unique chemical reactivity, because of strain associated with the three-membered ether ring. Various chemical conversions of the Formula (1) compounds are possible through ring opening reactions, and therefore, these compounds are important intermediates in the synthesis of organic compounds, and are also important as monomers in forming epoxy resins.

Stereoisomers of aliphatic epoxides can be used as raw materials in the synthesis of various compounds. Accordingly, if they could be analyzed, and also produced through a process that could be easily carried out on an industrial scale, a great contribution to the chemical arts would be made.

As conventional processes for obtaining optically active compounds, there are asymmetric synthesis methods, optical resolution methods by way of a diastereomer, and biochemical synthesis methods using an enzyme of a microorganism. Asymmetric synthesis processes have a drawback in that an intended compound having a high optical purity usually cannot be obtained. Similarly, processes which proceed by way of a diastereomer are difficult and equimolar amounts of different optically active compounds are necessary. Furthermore, biochemical synthesis processes are inadequate in that it is often quite difficult to find an appropriate enzyme or microorganism capable of achieving a desired synthesis.

A chiral separation of an aliphatic epoxide using a derivatized cyclodextrin as a chiral stationary phase (CSP) for a capillary gas chromatography column was reported by Professor Armstrong in <u>Anal. Chem.,</u> 1990, Volume 62, pp. 914-923 and in <u>A Guide to Using Cyclo-dextrin Bonded Phases for Gas Chromatography</u>, (1990 Edition), Astec Co. However, these methods did not provide a good separation that could be used for a trace analysis of one of the enantiomers. In addition, gas chromatography methods are only suitable for analytical purposes, instead of being applicable to the preparation of stereoisomers.

On the contrary, liquid and supercritical fluid based chromatography methods can be used for both analytical purposes and the preparation of stereoisomers. However, of known liquid (and supercritical fluid) chromatographic CSPs, none has been previously reported to be able to separate stereoisomers of an aliphatic epoxide, because the stereoisomers are believed to possess too little functionality to be separated.

Journal of Chromatography, 583 (1992), 231-235 describe the separation *cis*-epoxides of methyl and pentaflourobenzy esters of linoleic acid $\alpha$-linolenic acid, arachidonic acid and the $\omega$3-epoxide of eicosapentaenoic acid by normal-phase chromatography on a cellulose trisphenylcarbamate.

Chemical abstracts (1990) Vol.112: 775n (Anal. Biochem, Volume 182, no. 2,1989, 300-303) describe a chromatographic method for the direct enantiomeric characterization of all 4 regioisomeric epoxyeicosatrienoic acid metabolites generated by the cytochrome P 450 arachidonate epoxygenase pathway, wherein after esterification, the individual methyl or pentaflourobenzyl esters are resolved by chiral phase HPLC utilizing a Chiralcel® OB or CD column.

It is the object of the present invention to provide a method for the resolution of a mixture of stereoisomers of aliphatic epoxides by chromatographic techniques.

This object is achieved by a chromatographic method for resolving a mixture of stereoisomers of an aliphatic epoxide, the method comprising the steps of:
under chromatographic separating conditions, other than gas chromatographic separation conditions, passing said mixture with an eluent therefor into contact with an effective amount of a resolving agent comprising a polysaccharide or a derivative thereof for performing the resolution;
wherein the aliphatic epoxide has the following formula (I)

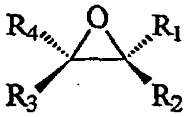

(I)

in which $R_1$ is different from $R_2$ and/or $R_3$ and/or $R_4$, and $R_1$, $R_2$, $R_3$ and $R_4$ are:

hydrogen; or
a saturated or an unsaturated aliphatic group having 1 to 30 carbon atoms selected from the group consisting of

an alkyl group, an alkenyl group having up to 5 double bonds and an alkynyl group having up to 5 triple bonds, and mixtures thereof, which aliphatic group may be optionally substituted by one or more substituents selected from the group consisting of a hydroxyl moiety, a halogenated moiety, and an ether moiety.

Preferred embodiments of the present invention are set forward in the claims.

The following Detailed Description, including the examples reported herein, is provided as an aid to those desiring to practice the present invention and is not to be construed as limiting to the scope of the inventor's discovery. In this respect the present invention is only to be limited by the scope of the claims appended hereto.

In Formula (1), the substituent $R_1$ is different from $R_2$ and/or $R_3$ and/or $R_4$ and the substituents $R_1$ to $R_4$ are selected from the group consisting of (1) hydrogen and (2) substituted or unsubstituted, saturated or unsaturated, aliphatic groups having 1 to 30 carbon atoms (exclusive of substituents), with acceptable saturated aliphatic groups including $C_{1-30}$ alkyl moieties, and acceptable unsaturated moieties including $C_{1-30}$ alkenylic moieties (having up to 5 double bonds) and $C_{1-30}$ alkynylic moieties (having up to 5 triple bonds), and mixtures thereof.

As specific examples of the $R_1$ to $R_4$ substituents that are present on the aliphatic epoxides of Formula (1) shown above there are mentioned -H, $-CH_3$, $-CH_2CH_3$,$-(CH_2)_nCH=CH_2$ (n=O-4), $-CH_2OH$, $-CH_2$-F, $-CH_2$-Cl, $-CH_2$-Br, $-OCH_3$, $-(CH_2)_nCH_3$ (n=3-25), and $-(CH_2)_4CH(CH_3)_2$.

The resolving agent used in the present invention comprises a polysaccharide or a derivative thereof, in an effective amount to effect a resolution of a mixture of stereoisomers encompassed by Formula (1). When the stereoisomers of Formula I which are being resolved are optically active compounds, the polysaccharide or derivative thereof is preferably optically active. Synthetic polysaccharides, natural polysaccharides and modified natural polysaccharides may be used in the inventive methods. Homoglycans having a constant bonding mode are preferred. Furthermore, it is preferred that a polysaccharide having a high purity be utilized which can be easily obtained. As preferred examples of a polysaccharide useful in the present invention, there are mentioned cellulose, amylose, β-1,4-chitosan, chitin, β-1,4-mannan, β-1,4-xylan, inulin, α-1,3-glucan, and β-1,3-glucan. When the chosen resolving agent is a polysaccharide derivative, a part or all, preferably at least 85%, of hydrogen atoms on hydroxyl groups of the polysaccharide (like one of the foregoing), are preferably replaced by other substituents, such as:

-C(O)R, -C(O)NH(R), -C(O)N(R)(R) and -R wherein R stands for an aliphatic group having 1 to 3 carbon atoms, an alicyclic group having 3 to 8 carbon atoms or an aromatic or heteroaromatic group having 4 to 20 carbon atoms.

The above-described types of polysaccharides and polysaccharide derivatives can be easily obtained by various known reactions. Moreover, these polysaccharides and their derivatives are especially suitable for chromatographic separation carried out on an industrial scale because they are easily available as starting materials and their stability is high.

Accordingly, in the chromatographic processes of the present invention, if an appropriate resolving agent is selected from the polysaccharides and derivatives thereof defined above, the stereoisomers of an aliphatic epoxide of Formula (1) can be resolved.

When liquid or supercritical fluid chromatography is adopted as a means for carrying out the method of the present invention, the chosen polysaccharide or its derivative is packed in a column directly or after it has been supported on a suitable carrier.

Since a granular resolving agent is preferred for liquid and supercritical chromatographic separation, when the polysaccharide or its derivative is used as the resolving agent, it is preferred that the polysaccharide or its derivative be pulverized or formed into beads. The particle size is variable according to the size of the column or plate used, but it is ordinarily 1μm to 10mm and preferable 1μm to 300μm. It is also preferred that the particles be porous.

In order to improve the pressure resistance of the resolving agent, prevent swelling or shrinkage by the solvent substitution and increase the number of theoretical stages, it is preferred that the polysaccharide or its derivative be supported on a carrier. The size of the carrier is variable according to the size of the column or plate used, but it is ordinarily 1μm to 10mm and preferably 1μm to 300μm. It is preferred that the carrier be porous, and the average pore size is 1 nm (10 Å) to 100μm, preferably 5 nm to 1 μm (50 Å to 10,000 Å), The amount of the polysaccharide or its derivative held on the carrier is ordinarily 1 to 1200% by weight and preferably 5 to 50% by weight based on the carrier.

Either a chemical method or a physical method may be used to support a polysaccharide or a derivative thereof on a carrier. As a physical method, there is mentioned (1) a method in which a polysaccharide or its derivative is dissolved in a solvent to form a solution, the solution is sufficiently mixed with a carrier and the solvent is distilled off under reduced pressure or by heating or by an air current, and (2) a method in which a polysaccharide or its derivative is dissolved in a solvent to form a solution, the solution is sufficiently mixed with a carrier and the mixture is stirred and then disbursed in a liquid having no compatibility with the solvent so as to diffuse the solvent. A heat treatment or the like may be performed so as to crystallize the polysaccharide or its derivative on the carrier. Moreover, there may be adopted a method in which a small amount of a solvent is added to swell or dissolve the polysaccharide or its derivative and the solvent is then distilled off to change the supported state and the resolving characteristics of the polysaccharide or its derivative.

A porous organic carrier and a porous inorganic carrier can be used, and a porous inorganic carrier is preferable. As preferred examples of the porous organic carrier, there are polymeric substances such as polystyrene, polyacrylamide and polyacrylate, and as preferred examples Of the porous inorganic carrier, there are synthetic and natural substances such as silica, alumina, magnesia, titanium oxide, glass, silicate and kaolin. A surface treatment may be carried out so as to improve the carrier's affinity with the polysaccharide or its derivative. As the surface treatment, there are mentioned silane treatments using an organic silane compound and plasma polymerization surface treatments.

When a polysaccharide or its derivative is used for the resolution, even in chemically identical derivatives, resolution characteristics can differ according to physical properties such as the molecular weight, the degree of crystallization and the orientation. Accordingly, the polysaccharide or its derivative may be subjected to a physical or chemical treatment such as a heat treatment or an etching treatment after giving a shape to the carrier suitable for the intended use, or during the step of giving a shape to the carrier suitable for the intended use.

In the case where thin layer chromatography is carried out when practicing the present inventive methods, a layer having a thickness of 0.1 to 100 mm, which is composed of particles of the resolving agent of the present invention having a size of about 0.1μm to about 0.1mm and, optically, a small amount of a binder, may be formed on a supporting plate.

As means for obtaining a pure isomer by using the above-mentioned resolving agent in the present invention, there are mentioned chromatographical processes such as liquid chromatography, a simulated moving bed adsorption system, supercritical fluid chromatography and thin layer chromatography. Gas chromatography is not a suitable means for obtaining a pure isomer utilizing the present inventive methods.

When practicing the present inventive methods and when liquid chromatography, supecritical fluid chromatography and thin layer chromatography are utilized, typical solvents may be used in addition to a solvent for the polysaccharide or the derivative thereof. If the resolving agent is chemically bonded to the carrier or is insolubilized by crosslinking, any liquids other than reactive liquids may be used without any limitations. Of course, the resolution characteristics, of a compound or an optical isomer vary according to the kind of the developing solvent, so that it is generally thought preferable and desirable to examine various solvents.

Utilizing the present invention, a stereoisomer of a compound represented by the general formula (1) which is an important industrial material, can be easily analyzed and obtained in a purified form, while using a cheap starting material and adopting a simple chemical conversion and a chromatographic technique. Accordingly, the inventive resolution methods make great contributions to the synthesis of many useful achiral and chiral compounds. That is, the invention provides a useful method of analyzing as well as producing stereoisomers of the aliphatic epoxides of Formula (1) shown above.

The present invention will now be described in detail with reference to the following examples that by no means limit the scope of the invention.

The liquid chromatography column utilized in the following Examples was a stainless column having a length of 25cm and an inner diameter of 0.46cm. It was packed with a filler which comprised silica gel treated with diphenylsilane and about 20 weight % of cellulose tris(3,5-dimethylphenylcarbamate) supported on the silica gel of 5μm (CHIRALCEL® OD-H). It was also packed with fillers which comprised silica gel treated with aminopropylsilane followed by 3,5-dimethylphenyl isocyanate and 20 weight % of amylose tris(3,5-dimethylphenylcarbamate) (CHIRALPAK® AD) or amylose tris((S)-methylbenzylcarbamate) (CHIRALPAK® AS) supported on the silica gel of 10μm (CHIRALCEL® and CHIRALPAK® are registered trademarks of Daicel Chemical Industries, Ltd.).

The stereoisomer eluted out was detected with a refractive index detector, ERMA ERC-7515A, a tradename of Erma CR.INC., an optical rotatory detector, SHODEX OR-1, a tradename of Showa Denko. A 880-PU, a tradename of JASCO was used as a liquid chromatography device with an injector, RHEODYNE 7125, a tradename of Rheodyne, and a degasser, ERC-3611, a tradename of Erma CR.INC.

The definitions of the terms used in the examples are as follows:

$$\text{Capacity factor } (k_n') = \frac{\text{(retention volume of peak number n) - (dead volume)}}{\text{(dead volume)}}$$

$$\text{Separation Factor } (\alpha) = \frac{\text{(capacity factor of more strongly retained isomer)}}{\text{(capacity factor of less strongly retained isomer)}}$$

$$\text{Resolution factor (Rs) } (n > m) = \frac{\text{(retention vol. of peak no. n) - (retention vol. of peak no. m)}}{\text{(sum of band widths of both peaks)/2}}$$

EXAMPLE 1

Separation on CHIRALCEL® OD-H

Table 1

| $R_1$ | $R_2$ | $R_3$ | $R_4$ | Eluent | $k_1'$ | Elution Order (faster/later) | α | Rs |
|---|---|---|---|---|---|---|---|---|
| -H | -H | -H | $-CH_2CH_3$ | A | 0.53 | +/- | >1.00* | - |
| -H | -H | -H | $-CH_2OH$ | A | 1.73 | +/- | >1.00* | - |
| -H | -H | -H | $-CH_2F$ | A | 1.01 | -/+ | >1.00* | - |
| -H | -H | -H | $-CH_2Cl$ | A | 0.96 | -/+ | >1.00* | - |
| -H | -H | -H | $-CH_2Br$ | A | 0.99 | -/+ | >1.00* | - |
| -H | -H | -H | $-CH_3$ | A | 0.58 | +/- | >1.00* | - |
| -H | $-CH_3$ | -H | $-CH_3$ | A | 3.75 | +/- | >1.00* | - |
| -H | -H | -H | $-(CH_2)_7CH_3$ | A | 0.41 | -/+ | >1.00* | - |
| -H | -H | -H | $-(CH_2)_9CH_3$ | A | 0.38 | -/+ | >1.00* | - |
| $-(CH_2)_9CH_3$ | -H | -H | $-(CH_2)_4CH(CH_3)_2$ | A | 0.28 | -/+ | >1.00* | - |
| -H | -H | -H | $-(CH_2)_{11}CH_3$ | A | 0.36 | -/+ | >1.00* | - |
| -H | -H | -H | $-(CH_2)_{13}CH_3$ | A | 0.34 | -/+ | >1.00* | - |
| -H | -H | -H | $-(CH_2)_{15}CH_3$ | A† | 0.32 | -/+ | >1.00* | - |
| $-CH_3$ | $-CH_3$ | -H | $-OCH_3$ | A | 0.54 | +/- | 1.12 | 1.31 |

EXAMPLE 1
Separation on CHIRALCEL® OD-H
Table 1 - Continued

Condition A:
Flow Rate: 1.0 ml/min
Temperature: Ambient
Volume of Injection: 0.2μl (neat)
Hexane / 2-Propanol = 95/5

* : Though α-value was not large enough to get a splitting peak by the refractive index detector, there was a clear indication of chiral separation by the optical rotatory detector.

† : 2 μl of 100 mg/ml sample was injected.

EXAMPLE 2
Separation on CHIRALPAK®AD
Table 2

| $R_1$ | $R_2$ | $R_3$ | $R_4$ | Eluent | $k_1'$ | Elution Order (faster/later) | α | Rs |
|---|---|---|---|---|---|---|---|---|
| -H | -H | -H | $-CH_2CH_3$ | B | 0.64 | +/- | 1.10 | 1.23 |
| -H | -H | -H | $-CH_2OH$ | B | 4.17 | -/+ | 1.26 | 4.13 |
| -H | -H | -H | $-CH_2F$ | B | 1.43 | -/+ | 1.10 | 1.78 |
| -H | -H | -H | $-CH_2Cl$ | B | 2.50 | -/+ | 1.42 | 4.06 |
| -H | -H | -H | $-CH_2Br$ | B | 3.66 | -/+ | 1.49 | 3.36 |
| -H | -H | -H | $-CH_3$ | A | 0.60 | +/- | 1.04 | - |
| -H | -H | -H | $-CH=CH_2$ | A | 0.62 | -/+ | >1.00* | - |
| $-CH_3$ | -H | -H | $-CH_3$ | A | 0.65 | ** | ** | ** |
| -H | $-CH_3$ | -H | $-CH_3$ | A | 0.62 | -/+ | >1.00* | - |
| -H | -H | -H | $-(CH_2)_3CH_3$ | A | 0.46 | -/+ | >1.00* | - |
| -H | -H | -H | $-(CH_2)_7CH_3$ | A | 0.33 | -/+ | >1.00* | - |
| -H | -H | -H | $-(CH_2)_9CH_3$ | A | 0.29 | -/+ | >1.00* | - |
| $-(CH_2)_9CH_3$ | -H | -H | $-(CH_2)_4CH(CH_3)_2$ | A | 0.23 | -/+ | >1.00* | - |
| -H | -H | -H | $-(CH_2)_{11}CH_3$ | A | 0.27 | -/+ | >1.00* | - |
| -H | -H | -H | $-(CH_2)_{13}CH_3$ | A | 0.34 | -/+ | >1.00* | - |

EXAMPLE 2

Separation on CHIRALPAK® AD

Table 2 - Continued

| $R_1$ | $R_2$ | $R_3$ | $R_4$ | Eluent | $k_1'$ | Elution Order (faster/later) | α | Rs |
|---|---|---|---|---|---|---|---|---|
| -H | -H | -H | $-(CH_2)_{15}CH_3$ | A† | 0.22 | -/+ | >1.00* | - |
| $-CH_3$ | $-CH_3$ | -H | $-OCH_3$ | A | 0.55 | -/+ | >1.00* | - |

Condition A:
Flow Rate: 1.0 ml/min
Temperature: Ambient
Volume of Injection: 0.2μl (neat)
Hexane/2-Propanol = 95/5

Condition B:
Flow Rate: 1.0 ml/min
Temperature: Ambient
Volume of Injection: 0.2 μl (neat)
Hexane/Ethanol = 90/10

* : Though α-value ws not large enough to get a splitting peak by the refractive index detector, there was a clear indication of chiral separation by the optical rotatory detector.

** : An achiral compound. It may be separated from its trans-isomer.

† : 2 μl of 100 mg/ml sample was injected.

EXAMPLE 3

Separation on CHIRALPAK®AS

Table 3

| $R_1$ | $R_2$ | $R_3$ | $R_4$ | Eluent | $k_1'$ | Elution Order (faster/later) | α | Rs |
|---|---|---|---|---|---|---|---|---|
| -H | -H | -H | $-CH_2CH_3$ | A | 0.62 | +/- | 1.14 | - |
| -H | -H | -H | $-CH_2OH$ | B | 2.01 | +/- | 1.04 | - |
| -H | -H | -H | $-CH_2F$ | A | 1.50 | -/+ | 1.11 | 1.19 |
| -H | -H | -H | $-CH_2Cl$ | A | 1.38 | -/+ | 1.15 | 1.56 |
| -H | -H | -H | $-CH_2Br$ | A | 1.41 | -/+ | 1.15 | 1.64 |
| -H | -H | -H | $-CH_3$ | A | 0.67 | +/- | 1.18 | 1.44 |
| -H | -H | -H | $-CH=CH_2$ | C | 1.20 | -/+ | 1.17 | 0.67 |
| $-CH_3$ | -H | -H | $-CH_3$ | A | 0.66 | ** | ** | ** |
| H | $-CH_3$ | -H | $-CH_3$ | A | 0.54 | +/- | 1.58 | 3.47 |
| -H | -H | -H | $-(CH_2)_3CH_3$ | A | 0.53 | +/- | 1.25 | 1.94 |
| -H | -H | -H | $-(CH_2)_7CH_3$ | C | 0.50 | +/- | 1.26 | 1.68 |
| -H | -H | -H | $-(CH_2)_9CH_3$ | D | 0.40 | +/- | 1.24 | 1.28 |

Example 3
Separation on CHIRALPAK® AS
Table 3 - Continued

| $R_1$ | $R_2$ | $R_3$ | $R_4$ | Eluent | $k_1'$ | Elution Order (faster/later) | α | Rs |
|---|---|---|---|---|---|---|---|---|
| -H | -H | -H | $-(CH_2)_2CH{=}CH_2$ | A | 0.71 | +/- | 1.26 | 2.15 |
| $-(CH_2)_9CH_3$ | -H | -H | $-(CH_2)_4CH(CH_3)_2$ | A | 0.23 | -/+ | >1.00* | - |
| -H | -H | -H | $-(CH_2)_{11}CH_3$ | D | 0.36 | +/- | 1.22 | 1.40 |
| -H | -H | -H | $-(CH_2)_{13}CH_3$ | D | 0.32 | -/+ | 1.20 | - |
| -H | -H | -H | $-(CH_2)_{15}CH_3$ | A† | 0.29 | -/+ | 1.15 | - |
| $-CH_3$ | $-CH_3$ | -H | $-OCH_3$ | A | 0.52 | +/- | 1.07 | - |

Condition A:
Flow Rate: 1.0 ml/min
Temperature: Ambient
Volume of Injection: 0.2 µl (neat)
Hexane/2-Propanol = 95/5

Conditon B:
Flow Rate: 1.0 ml/min
Temperature: Ambient
Volume of Injection: 0.2 µl (neat)
Hexane/Ethanol = 90/10

Condition C:
Flow Rate: 1.0 ml/min
Temperature: -10°C
Volume of Injection: 0.2 µl (neat)
Hexane/2-Propanol = 95/5

Condition D:
Flow Rate: 1.0 ml/min
Temperature: Ambient
Volume of Injection: 0.2 µl (neat)
Hexane/2-Propanol = 99/1

* : Though α-value was not large enough to get a splitting peak by the refractive index detector, there was a clear indication of chiral separation by the optical rotatory detector.

** : An achiral compound. It may be separated from its trans-isomer.

† : 2 $\mu l$ of 100 mg/ml sample was injected.

The invention being thus described, it will be obvious that the same may be varied in many ways. Such variations are not to be regarded as a departure from the scope of the invention, and all such modifications as would be obvious to one skilled in the art are intended to be included within the scope of the following claims.

## Claims

1. A chromatographic method for resolving a mixture of stereoisomers of an aliphatic epoxide, the method comprising the steps of:

under chromatographic separating conditions, other than gas chromatographic separation conditions, passing said mixture with an eluent therefor into contact with an effective amount of a resolving agent comprising a polysaccharide or a derivative thereof for performing the resolution;
wherein the aliphatic epoxide has the following formula (I)

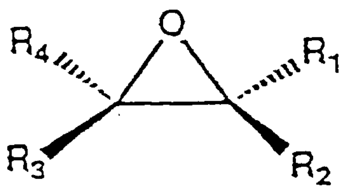

(I)

in which $R_1$ is different from $R_2$ and/or $R_3$ and/or $R_4$, and $R_1$, $R_2$, $R_3$ and $R_4$ are:

hydrogen; or

a saturated or an unsaturated aliphatic group having 1 to 30 carbon atoms selected from the group consisting of an alkyl group, an alkenyl group having up to 5 double bonds and an alkynyl group having up to 5 triple bonds, and mixtures thereof, which aliphatic group may be optionally substituted by one or more substituents selected from the group consisting of a hydroxyl moiety, a halogenated moiety, and an ether moiety.

**2.** The method of claim 1, wherein the polysaccharide or the derivative thereof is optically active.

**3.** The method of claim 1 or 2, wherein in the compound of formula (I), $R_1$ to $R_4$ are selected from the group consisting of:
$-H$, $-CH_3$, $-CH_2CH_3$, $-(CH_2)_nCH=CH_2$ (n=0-4), $-CH_2OH$, $-CH_2-F$, $-CH_2-Cl$, $-CH_2-Br$, $-OCH_3$, $-(CH_2)_nCH_3$ (n = 3-25) and $-(CH_2)_4-CH(CH_3)_2$.

**4.** The method of claim 1 or 2, wherein in the compound of formula (I) $R_1$, $R_2$ and $R_3$ are each hydrogen and $R_4$ is $-CH_2OH$:

**5.** The method of claim 1 or 2, wherein in the compound of formula (I) $R_1$, $R_2$ and $R_3$ are each hydrogen and $R_4$ is $-CH_2F$.

**6.** The method of claim 1 or 2, wherein in the compound of formula (I) $R_1$, $R_2$ and $R_3$ are each hydrogen and $R_4$ is $-CH_2Cl$.

**7.** The method of claim 1 or 2, wherein in the compound of formula (I) $R_1$, $R_2$ and $R_3$ are each hydrogen and $R_4$ is $-CH_2Br$.

**8.** The method of claim 1 or 2, wherein in the compound of formula (I) $R_1$, $R_2$ and $R_3$ are each hydrogen and $R_4$ is $-CH_2CH_3$.

**9.** The method of any of claims 1 to 8, wherein the polysaccharide or derivative thereof is a homoglycan having a constant bonding mode.

**10.** The method of any of claims 1 to 8, wherein the polysaccharide is selected from the group consisting of:
cellulose, amylose, β-1,4-chitosan, chitin, β-1,4-mannan, β-1,4-xylan, inulin, α-1,3-glucan, and β-1,3-glucan.

**11.** The method of any of claims 1 to 10, wherein the polysaccharide derivative is a derivative of a polysaccharide wherein at least a part of the hydrogen atoms on hydroxyl groups of the polysaccharide are replaced by a substituent selected from the group consisting of:
-C(O)R, -C(O)NH(R), -C(O)N(R)(R) and -R,
wherein R is an aliphatic group having 1 to 3 carbon atoms, an alicyclic group having 3 to 8 carbon atoms or an aromatic or heteroaromatic group having 4 to 20 carbon atoms.

**12.** The method of claim 11, wherein the polysaccharide derivative is a derivative of a polysaccharide wherein at least

85% of the hydrogen atoms on hydroxyl groups of the polysacharide are replaced by said substituents.

**13.** The method of any of claims 1 to 12, wherein said resolving agent is cellulose tris(3,5-dimethylphenylcarbamate) supported on silica gel.

**14.** The method of any of claims 1 to 12, wherein said resolving agent is amylose tris((S)-methylbenzylcarbamate) supported on silica gel.

**15.** The method of any of claims 1 to 12, wherein said resolving agent is amylose tris(3,5-dimethylphenylcarbamate) supported on silica gel.

**16.** The method of any of claims 1 to 15, wherein said chromatographic method is a simulated moving bed adsorption system.

**17.** The method of any of claims 1 to 15, wherein said chromatographic method is a supercritical fluid chromatography method.

**Patentansprüche**

**1.** Chromatographisches Verfahren zum Trennen einer Mischung von Stereoisomeren eines aliphatischen Epoxids, wobei des Verfahren die Schritte umfaßt, daß man

- unter chromatographischen Trennbedingungen, die von gaschromatographischen Trennbedingungen verschieden sind, die Mischung mit einem Eluens hierfür zur Durchführung der Trennung in Kontakt mit einer wirksamen Menge eines trennenden Mittels bringt, das ein Polysaccharid oder ein Derivat davon umfaßt;
- wobei das aliphatische Epoxid die folgende Formel (I) aufweist:

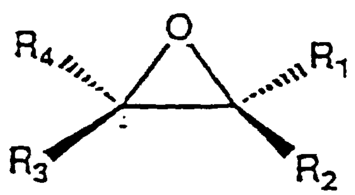

worin $R_1$ von $R_2$ und/oder $R_3$ und/oder $R_4$ verschieden ist und $R_1$, $R_2$, $R_3$ und $R_4$ stehen für Wasserstoff; oder
eine gesättigte oder eine ungesättigte aliphatische Gruppe mit 1 bis 30 Kohlenstoffatomen, die gewählt ist aus der Gruppe, die besteht aus einer Alkyl-Gruppe, einer Alkenyl-Gruppe mit bis zu 5 Doppelbindungen und einer Alkinyl-Gruppe mit bis zu 5 Dreifachbindungen und Mischungen daraus, wobei die aliphatische Gruppe gegebenenfalls substituiert sein kann durch einen oder mehrere Substituenten, der/die gewählt ist/sind aus der Gruppe, die besteht aus einer Hydroxyl-Einheit, einer halogenierten Einheit und einer Ether-Einheit.

**2.** Verfahren nach Anspruch 1, worin das Polysaccharid oder dessen Derivat optisch aktiv ist.

**3.** Verfahren nach Anspruch 1 oder 2, worin in der Verbindung der Formel (I) $R_1$ bis $R_4$ gewählt sind aus der Gruppe, die besteht aus:
$-H$, $-CH_3$, $-CH_2CH_3$, $-(CH_2)_nCH=CH_2$ (n = 0 bis 4), $-CH_2OH$, $-CH_2$-F, $-CH_2$-Cl, $-CH_2$-Br, $-OCH_3$, $-(CH_2)_nCH_3$ (n = 3 bis 25) und $-(CH_2)_4$-CH-$(CH_3)_2$.

**4.** Verfahren nach Anspruch 1 oder 2, worin in der Verbindung der Formel (I) $R_1$, $R_2$ und $R_3$ jeweils Wasserstoff sind und $R_4$ für $-CH_2OH$ steht.

**5.** Verfahren nach Anspruch 1 oder 2, worin in der Verbindung der Formel (I) $R_1$, $R_2$ und $R_3$ jeweils Wasserstoff sind und $R_4$ für $-CH_2F$ steht.

**6.** Verfahren nach Anspruch 1 oder 2, worin in der Verbindung der Formel (I) $R_1$, $R_2$ und $R_3$ jeweils Wasserstoff sind

und $R_4$ für $-CH_2Cl$ steht.

**7.** Verfahren nach Anspruch 1 oder 2, worin in der Verbindung der Formel (I) $R_1$, $R_2$ und $R_3$ jeweils Wasserstoff sind und $R_4$ für $-CH_2Br$ steht.

**8.** Verfahren nach Anspruch 1 oder 2, worin in der Verbindung der Formel (I) $R_1$, $R_2$ und $R_3$ jeweils Wasserstoff sind und $R_4$ für $-CH_2CH_3$ steht.

**9.** Verfahren nach einem der Ansprüche 1 bis 8, worin das Polysaccharid oder dessen Derivat ein Homoglykan ist, das eine konstante Bindungsart aufweist.

**10.** Verfahren nach einem der Ansprüche 1 bis 8, worin das Polysaccharid gewählt ist aus der Gruppe, die besteht aus: Cellulose, Amylose, β-1,4-Chitosan, Chitin, β-1,4-Mannan, β-1,4-Xylan, Inulin, α-1,3-Glukan und β-1,3-Glukan.

**11.** Verfahren nach einem der Ansprüche 1 bis 10, worin das Polysaccharid-Derivat ein Derivat eines Polysaccharids ist, in dem wenigstens ein Teil der Wasserstoffatome an den Hydroxyl-Gruppen des Polysaccharids durch einen Substituenten ersetzt sind, der gewählt ist aus der Gruppe, die besteht aus:

-C(O)R, -C(O)NH(R), -C(O)N(R)(R) und -R,

worin R für eine aliphatische Gruppe mit 1 bis 3 Kohlenstoffatomen, eine alicyclische Gruppe mit 3 bis 8 Kohlenstoffatomen oder eine aromatische oder heteroaromatische Gruppe mit 4 bis 20 Kohlenstoffatomen steht.

**12.** Verfahren nach Anspruch 11, worin das Polysaccharid-Derivat ein Derivat eines Polysaccharids ist, in dem wenigstens 85 % der Wasserstoffatome an den Hydroxyl-Gruppen des Polysaccharids durch die Substituenten ersetzt sind.

**13.** Verfahren nach einem der Ansprüche 1 bis 12, worin das trennende Mittel Cellulose-tris (3,5-dimethylphenylcarbamat) auf Silicagel als Träger ist.

**14.** Verfahren nach einem der Ansprüche 1 bis 12, worin das trennende Mittel Amylose-tris ((S)-methylbenzylcarbamat) auf Silicagel als Träger ist.

**15.** Verfahren nach einem der Ansprüche 1 bis 12, worin das trennende Mittel Amylose-tris (3,5-dimethylphenylcarbamat) auf Silicagel als Träger ist.

**16.** Verfahren nach einem der Ansprüche 1 bis 15, worin das chromatographische Verfahren ein simuliertes Bewegtbett-Adsorptionssystem ist.

**17.** Verfahren nach einem der Ansprüche 1 bis 15, worin das chromatographische Verfahren ein Chromatographie-Verfahren mit einem überkritischen Fluid ist.

**Revendications**

**1.** Procédé chromatographique pour résoudre un mélange de stéréoisomères d'un époxyde aliphatique, ledit procédé comprenant les étapes suivantes :

dans des conditions de séparation chromatographique, autres que des conditions de séparation chromatographique en phase gazeuse, faire passer ledit mélange avec un éluant pour celui-ci en contact avec une quantité efficace d'un agent de résolution comprenant un polysaccharide ou un dérivé de celui-ci pour effectuer la résolution ;
ledit époxyde aliphatique ayant la formule (I) suivante

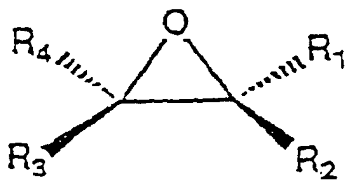

(I)

dans laquelle $R_1$ est différent de $R_2$ et/ou $R_3$ et/ou $R_4$, et $R_1$, $R_2$, $R_3$ et $R_4$ sont :

un atome d'hydrogène ; ou

un groupe aliphatique saturé ou insaturé ayant 1 à 30 atomes de carbone, choisi dans la classe formée par un groupe aikyle, un groupe alcényle ayant jusqu'à 5 doubles liaisons et un groupe alcynyle ayant jusqu'à 5 triples liaisons, et leurs mélanges, ce groupe aliphatique pouvant facultativement être substitué par un ou plusieurs substituants choisis dans la classe formée par un radical hydroxyle, un radical halogéné et un radical éther.

**2.** Procédé selon la revendication 1, dans lequel le polysaccharide ou son dérivé est optiquement actif.

**3.** Procédé selon la revendication 1 ou 2, dans lequel, dans le composé de formule (I), $R_1$ à $R_4$ sont choisis dans la classe formée par
$-H$, $-CH_3$, $-CH_2CH_3$, $-(CH_2)_nCH=CH_2$ (n = 0 à 4), $-CH_2OH$,
$-CH_2-F$, $-CH_2-Cl$, $-CH_2-Br$, $-OCH_3$, $-(CH_2)_nCH_3$ (n = 3 à 25)
et - $(CH_2)_4-CH(CH_3)_2$.

**4.** Procédé selon la revendication 1 ou 2, dans lequel, dans le composé de formule (I), $R_1$, $R_2$ et $R_3$ sont chacun un atome d'hydrogène et $R_4$ est $-CH_2OH$.

**5.** Procédé selon la revendication 1 ou 2, dans lequel, dans le composé de formule (I), $R_1$, $R_2$ et $R_3$ sont chacun un atome d'hydrogène et $R_4$ est $-CH_2F$.

**6.** Procédé selon la revendication 1 ou 2, dans lequel, dans le composé de formule (I), $R_1$, $R_2$ et $R_3$ sont chacun un atome d'hydrogène et $R_4$ est $-CH_2Cl$.

**7.** Procédé selon la revendication 1 ou 2, dans lequel, dans le composé de formule (I), $R_1$, $R_2$ et $R_3$ sont chacun un atome d'hydrogène et $R_4$ est $-CH_2Br$.

**8.** Procédé selon la revendication 1 ou 2, dans lequel, dans le composé de formule (I), $R_1$, $R_2$ et $R_3$ sont chacun un atome d'hydrogène et $R_4$ est $-CH_2CH_3$.

**9.** Procédé selon l'une quelconque des revendications 1 à 8, dans lequel le polysaccharide ou Son dérivé est un homoglycane ayant un mode de liaison constant.

**10.** Procédé selon l'une quelconque des revendications 1 à 8, dans lequel le polysaccharide est choisi dans la classe formée par :
la cellulose, l'amylose, le β-1,4-chitosane, la chitine, le β-1,4-mannane, le β-1,4-xylane, l'inuline, le α-1,3-glucane et le β-1,3-glucane.

**11.** Procédé selon l'une quelconque des revendications 1 à 10, dans lequel le dérivé de polysaccharide est un dérivé d'un polysaccharide dans lequel les atomes d'hydrogène des groupes hydroxyle du polysaccharide sont au moins partiellement remplacés par un substituant choisi dans la classe formée par :
-C(O)R, -C(O)NH(R), -C(O)N(R)(R) et -R,
où R est un groupe aliphatique ayant 1 à 3 atomes de carbone, un groupe alicyclique ayant 3 à 8 atomes de carbone ou un groupe aromatique ou hétéroaromatique ayant 4 à 20 atomes de carbone.

**12.** Procédé selon la revendication 11, dans lequel le dérivé de polysaccharide est un dérivé d'un polysaccharide dans lequel au moins 85 % des atomes d'hydrogène des groupes hydroxyle du polysaccharide sont remplacés par

lesdits substituants.

13. Procédé selon l'une quelconque des revendications 1 à 12, dans lequel ledit agent de résolution est le tris(3,5-diméthylphénylcarbamate) de cellulose supporté par un gel de silice.

14. Procédé selon l'une quelconque des revendications 1 à 12, dans lequel ledit agent de résolution est le tris((S)-méthylbenzylcarbamate) d'amylose supporté par du gel de silice.

15. Procédé selon l'une quelconque des revendications 1 à 12, dans lequel ledit agent de résolution est le tris(3,5-diméthylphénylcarbamate) d'amylose supporté par du gel de silice.

16. Procédé selon l'une quelconque des revendications 1 à 15, dans lequel ledit procédé chromatographique est un système d'adsorption en lit mobile simulé.

17. Procédé selon l'une quelconque des revendications 1 à 15, dans lequel ledit procédé chromatographique est un procédé de chromatographie à fluide surcritique.